(19) Europäisches Patentamt
European Patent Office
Office européen des brevets

(11) Publication number: **0 406 880 A2**

# (12) EUROPEAN PATENT APPLICATION

(21) Application number: **90112928.8**

(22) Date of filing: **06.07.90**

(51) Int. Cl.5: **A61K 39/395**

The application is published incomplete as filed (Article 93 (2) EPC). The point in the description (pages 33 and 34 of the originally filed description) at which the omission obviously occurs has been left blank.

A request for addition has been filed pursuant to Rule 88 EPC. A decision on the request will be taken during the proceedings before the Examining Division (Guidelines for Examination in the EPO, A-V, 2.2).

(30) Priority: **07.07.89 US 376704**
**07.09.89 US 404266**
**28.06.90 US 543442**

(43) Date of publication of application:
**09.01.91 Bulletin 91/02**

(84) Designated Contracting States:
**AT BE CH DE DK ES FR GB GR IT LI LU NL SE**

(71) Applicant: **THE UNIVERSITY OF KANSAS**
**c/o Office of Research and Graduate Studies, Rainbow Boulevard, 39th Street**
**Kansas City, Kansas 66183(US)**

(72) Inventor: **Morrison, David C.**
**Rte. 1, Box 128**
**Kearney, MO 64060(US)**
Inventor: **Chen, Taiying**
**5200 Hadley Court 4**
**Overland Park Kansas 66202(US)**
Inventor: **Lei, Mei-Guey**
**9144 West 91st Street Terrace**
**Overland Park Kansas 66212(US)**
Inventor: **Bright, Stuart W.**
**9511 East 64th Street**
**Raytown, MO 64133(US)**
Inventor: **Flebbe, Linda M.**
**608 E. 101 Street, 202**
**Kansas City, MO 64131(US)**

(74) Representative: **Patentanwälte Schulze Horn und Hoffmeister**
**Goldstrasse 36**
**D-4400 Münster(DE)**

## (54) Method for stimulating macrophage activity against tumors and composition therefor.

(57) It has been found that monoclonal antibodies which specifically bind to the protein portion of the 80 kilodalton glycoprotein receptor for bacterial lipopolysaccharide endotoxin, 5D3 in particular, stimulate the cytotoxic effect of macrophages against tumors, such as plasmocytomas. The effect is potentiated by combination with gamma interferon. Also described are compositions containing the monoclonal antibody and gamma interferon.

EP 0 406 880 A2

# METHOD FOR STIMULATING MACROPHAGE ACTIVITY AGAINST TUMORS AND COMPOSITION THEREFOR

## RELATED APPLICATIONS

This application is a continuation in part of U.S. patent application Serial Number 404,266 filed September 7, 1989, which is itself a continuation-in-part of U.S. patent application Serial No. 376,704, filed July 7, 1989.

## FIELD OF THE INVENTION

This invention relates to uses of monoclonal antibodies which specifically bind to the protein component of purified bacterial lipopolysaccharide ("LPS" hereafter) binding factor on mammalian cells ("receptor" hereafter), and compositions containing these monoclonal antibodies. The uses include macrophage activation, with resulting mediation of tumor cytotoxicity, as well as acting as an agonist against the LPS receptor in dealing with the effects of endotoxin shock.

## BRIEF DESCRIPTION OF THE PRIOR ART

The bacteria that can cause bacteremia, sepsis, and the septic-shock syndrome are usually part of the body's normal bacterial flora. If their numbers are kept in check, they are harmless. However in certain subsets of patients the bacteria have a prime environment in which to multiply. These include patients with suppressed immune systems, who receive continuous corticosteroid therapy or radiotherapy, the elderly, post-surgical patients, patients with such chronic diseases as diabetes and liver disease, patients with severe burns, and infants with meningococcal infections. If, in these patients, bacteria leak into the bloodstream through the effects of surgery or catheterization or by other means, septic shock is initiated.

There is strong evidence that endotoxins, lipopolysaccharide-protein complexes found in the cell walls of gram-negative bacteria, lead to the course of the septic shock. Researchers theorize that the endotoxins, released by gram-negative bacteria, bind to specific receptor sites on cell membranes of host target cells. The toxins initiate the production of numerous mediators, including tumor necrosis factor (TNF)/cachectin, interleukins 1, 2 and 6, interferon, C3, procoagulant activity (tissue factor activity), as well as the prostaglandins and leukotrienes, and platelet activating factor. Symptoms which appear as the body tries to rid itself of the excess bacteria and/or endotoxin include fever, shock, disseminated intravascular coagulation, complement activation, transient leukopenia followed by leukocytosis, multisystem organ failure and death.

There are about 200,000 cases of septic shock per year in the United States, 80% of which are caused by gram-negative organisms.

Treatment for septic shock, a syndrome that can kill a patient in 48 hours, typically consists of a combination of antibiotics, vasopressors, and inotropes. A narçotic antagonist, Naloxone (Narcan, Dupont) is also used. However, the results so far are only for a few patients and none have shown the agent to be efficacious (Lancet 2: 699-702, 1988).

There remains a need for an effective agent that can be used to help standardize and improve treatment.

Any attempt to neutralize the endotoxin encounters the problem of the multiple chemical form of endotoxins, approximately 1,000 to 10,000 which may exist. Even though these toxins are similar in structure, they are usually not sufficiently similar to react with the major type of antibodies made against these toxins. The same problem may exist in obtaining mediator-directed monoclonal antibodies, since many mediators may contribute and multiple interdependent pathways exist for their production and/or amplification.

The use of special monoclonal antibodies to counter endotoxin is possible based upon a very limited cross-reactivity with some structural features which are conserved among endotoxins. Such antibodies might neutralize the endotoxin or block the endotoxin site which binds to the receptor or react with the cell products which cause the shock such as TNF.

The above identified parent and grandparent applications, the disclosures of which are incorporated by reference, teach the production of monoclonal antibodies which specifically bind to the LPS binding factor (receptor). This receptor is characterized as a glycoprotein of about 80 kilodalton molecular weight, and via the functional properties described supra . In particular monoclonal antibodies ("mAb") 5D3 and 3D7 are described, which bind to protein and carbohydrate components of the receptor, respectively. Both monoclonal antibodies have been deposited at the American Type Culture Collection.

Among the properties of the described mAbs are the ability to prevent the binding of LPS to the receptor, thereby inhibiting the normal consequence of this complexing. The results in the parent and grandparent application, also presented herein, show the efficacy of the mAbs as in vivo anti-toxic shock agents.

It has now been found that the monoclonal antibodies directed against the protein component of the receptor have additional properties not to be expected from the results obtained before. Specifically, it has been found that these mAbs, alone or in combination with, e.g., gamma interferon ("IFN-$\gamma$") induce macrophage-mediated tumor cytotoxicity, and also function as agonists to the receptor, both in vivo and in vitro.

## DESCRIPTION OF THE FIGURES

Figure 1 shows the capacity of mAb 3D7 to inhibit the binding of endotoxic LPS to the 80 KD LPS receptor.

Figure 2 shows inhibition of binding of mAb 3D7 to splenocytes by LPS.

Figure 3 show mitogenicity of purified MoAb 5D3.

Figure 4 shows the reactivity of MoAb 5D3 to purified 80 KD LPS binding protein.

Figure 5 shows the reactivity of rabbit polyclonal-antimonoclonal antisera against MoAb 5D3 (5a) and pre B cell 70Z/3 (5b).

Figure 6 shows Western blot and immunoprecipitation of rabbit polyclonal-antimonoclonal antisera against MoAb 5D3 and 702/3 pre B cels.

Figure 7 presents data showing the effect of mAb 5D3 and LPS from E. coli on bone marrow derived macrophage mediated tumor cytotoxicity.

Figure 8 depicts the effect of mAb 5D3 on LPS responder and nonresponder derived macrophages.

Figure 9 shows the effect of heating and polymyxin B on mAb 5D3 activation of macrophages.

Figure 10 shows the effect of E. coli LPS and mAb 5D3 activity, in terms of macrophage mediated tumor cytotoxicity.

Figure 11 shows the effect of anti IFN $\alpha/\beta$ antibodies on the activity of mAb 5D3, in terms of macrophage mediated tumor cytotoxicity.

## DETAILED DESCRIPTION OF THE INVENTION

Monoclonal antibodies specific for bacterial lipopoly saccharide endotoxin-binding receptor on mammalian cells (hereinafter "LPS receptor") are produced by antibody-producing cell lines which may be hybrid cell lines commonly known as hybridomas. The hybrid cell are formed by the fusion of an anti-LPS receptor antibody producing cell and an immortalized cell line; that is a cell line which imparts long term tissue culture stability of the hybrid cell. In the formation of the hybrid cell lines, the first fusion partner - the anti-LPS receptor antibody producing cell -may be a spleen cell from an animal immunized with an LPS receptor positive B cell, or a biochemical preparation enriched for the LPS receptor. The second fusion partner -the immortal cell - may be a lymphoblastoid cell or a plasmacytoma cell such as a myeloma cell, itself a precursor of an antibody producing cell selected so as to be a non-antibody secretor but also malignant.

Rodent hybridomas which produce LPS receptor specific monoclonal antibodies are formed by the fusion of rodent myeloma cells and spleen cells from rodents immunized against LPS receptor positive B cells, purified LPS receptor, or other biological preparations comprising LPS receptor. To immunize the rodents, a variety of different protocols may be followed. For example, rodents may receive primary and boosting immunizations of LPS receptor positive B cells or partially purified LPS receptor. The fusions are accomplished by standard procedures well known to those skilled in the field of immunology. Kohler and Milstein, Nature , 256 : 495-497 (1975) and Kennet, Monoclonal Antibodies (Kennet et al., Eds. pp. 365-367, Plenum Press, N.Y. 1980).

The resulting clones are then screened for production of antibody reactive with LPS receptor positive B cells or biological preparations comprising LPS receptor. Those which secrete reactive antibodies are cloned.

Human hybridomas which produce monoclonal anti-LPS receptor antibodies are formed from the fusion of spleen cells from an individual producing anti-LPS receptor antibodies and a human lymphoblastoid cell line. Alternatively, the fusion partner for the myeloma cell may be a peripheral blood anti-LPS receptor producing lymphocyte. The fusion and screening techniques are essentially the same as those used in the production and selection of hamster anti-LPS receptor generating hybridomas.

Another way of forming the anti-LPS receptor producing cell line is by transformation of antibody producing cells. For example, an anti-LPS receptor producing B lymphocyte may be infected and transformed with a virus such as Epstein-Barr virus in the case of B lymphocytes to yield an immortal anti-LPS receptor producing cell. See e.g., Kozbor and Roder, Immunology Today , 4(3) : 72-79 (1983). Alternatively the B lymphocyte may be trans-formed by a transforming gene or transforming gene product.

The LPS receptor specific monoclonal antibodies are produced in large quantities by injecting anti-LPS receptor antibody producing hybridomas into the peritoneal cavity of rodents and, after appropriate time, harvesting the ascites fluid which contains a high titer of homogeneous antibody and isolating the monoclonal anti-LPS receptor antibody therefrom. Xenogenic hybridomas should be injected into irradiated or athymic nude mice. Alternatively, the antibodies may be produced by culturing anti-LPS receptor producing cells *in vitro* and isolating secreted monoclonal anti-LPS receptor antibodies from the cell culture medium.

Chimeric anti-LPS receptor antibodies are produced by cloning DNA segments encoding the heavy and light chain variable regions of a non-human antibody specific for LPS receptor and joining these DNA segments to DNA segments encoding human heavy and light chain constant regions to produce chimeric immunoglobulin encoding genes. The fused gene constructs coding for the light and heavy chains are assembled in or inserted into expression vectors. The genes are cotransfected into a lymphoid recipient cell (e.g., a myeloma cell) where the immunoglobulin protein can be synthesized, assembled and secreted. The transfected recipients cells are cultured and the expressed immunoglobulins are collected.

## EXAMPLE 1

The 80 kD LPS-binding protein, isoelectric point 6.5 has been partially purified from mouse splenocytes [T-Y Chen et al. FASEB Journal 3 No. 4 A 1082, #4969 1989] saturated with radioiodinated photo-activatable bacterial LPS endotoxin derivative cross-linked by UV irradiation. Cells were sonicated and subjected to a sucrose gradient. An LPS binding protein enriched fraction was treated with aqueous butanol at 4° C and the biphasic mixture separated by centrifugation, the receptor-LPS complex partitions in the aqueous phase. After dialysis and concentration, the aqueous extract was reduced and fractionated by preparative SDS-PAGE.

The single radiolabelled band was electroeluted from the gel and when rerun on two-dimensional polyacrylamide gel, yielded a single radiolabelled spot. The amino acid composition of this protein is shown in Table 1.

Characterization, amino acid sequencing and molecular cloning of the cDNA for this receptor are now possible. Determination of the active binding site will allow for the design and synthesis of active receptor site analogues which will either block the ability of LPS to bind to the receptor or bind competitively to the active site without effecting cell activation.

## EXAMPLE 2

The LPS binding protein receptor from example 1 above was excised from the gel, homogenized in adjuvant and used to immunize three month old Armenian hamsters.

### Cells and tissue culture medium

The cell line Sp2/0-Ag14 (Sp2) was used as a fusion partner. Splenocytes from the LPS binding protein receptor immunized Armenian hamsters were used as a source of antibody producing cells. A single cell suspension of splenocytes was obtained by teasing a sterile hamster spleen through a stainless steel mesh using a plunger from a 3cc syringe. The splenocyte suspension and Sp2 cells were washed individually with culture medium (with no Fetal Calf Serum) and collected by centrifugation (500 x g, 10 min). The basal medium used was RPMI 1640 supplemented with glutamine (4mM), Penicillin-Streptomycin (2%), Non-essential amino acids (1%), Sodium Pyruvate (1 mM) and Fetal Calf Serum (15%).

## Animals

Female Armenian Hamsters (Cytogen), 12 week-old, were used for immunization.

## Antigen

Protein eluted from the 80 kDa portion of an SDS gel loaded with a LPS binding protein enriched fraction from lysed murine splenocytes.

## Immunization

Hamsters were immunized with a subcutaneous injection of 10 ug of antigen in 250 ul of PBS emulsified in an equal volume of complete Freund's adjuvant. The hamsters were boosted after 14 days with 10 ug of adjuvant in 250 ul of PBS and 250 ul of incomplete Freund's adjuvant. Another boost was administered at day 28 using 20 ug of antigen in incomplete Freund's adjuvant. A final boost of 20 ug of antigen in 500 ul PBS was injected intraperitoneally on day 64, three days before cell fusion.

## Fusion

One hamster spleen yielded approximately 7.5 x $10^7$ splenocytes. The washed splenocytes were pooled and centrifuged with 1.5 x $10^7$ Sp2/0 cells. To the sedimented cells in a 50 mL conical tube, 1 mL of 50% PEG 1450 (in RPMI 1640) was added over 2 minutes. The cell suspension was diluted dropwise with 1 mL media without serum over minute 3, 2 mLs at minute 4, 4 mls at minute 5 and 8 mls at minute 6. The suspension was pelleted immediately (500 x g, 5 min) and resuspended in basal medium supplemented with FCS and HAT and dispensed on 96 well plates at 1 x $10^5$ viable cells/100ul/well.

## Screening for positive hybridomas

Culture supernatants from each well were screened by ELISA on whole 70Z/3 cells and partially purified 80 kDa LPS receptor antigen at 5 ug/mL. Bound hamster antibodies were detected with peroxidase-labeled rabbit anti-hamster Ig plus TMB (tetra-methyl-benzidine) as substrate. Optical density was read at 430 nm.

Supernatants positive for the 80 kDa LPS receptor antigen were screened with one-dimensional western blots.

## Subcloning

Hybridomas of interest were subcloned in soft agar using 0.6% agarose (SeaPrep, FMC) in supplemented medium.

Two monoclonal antibodies (3D7 and 5D3) were isolated following the immunization protocol (subcutaneous immunization). The monoclonals (3D7 and 5D3) are IgM and 19S molecular size. These two monoclonal antibodies are directed against two different epitopes of the 80 kD LPS-binding receptor glycoprotein. One is directed against the carbohydrate moiety and the other against the protein determinant. (Table 2). Preliminary results indicate inhibition of mouse lethality by monoclonal 5D3 antibody pretreatment (Table 3).

The use of monoclonal antibody and/or components of the antibody (e.g. Fab) to the endotoxin receptor can be employed to compete with endotoxin for binding to the receptor and thus inhibit white blood cell or endothelial cell activation. Alternatively the monoclonal can be employed as an immunogen to prepare antiidiotype antibody which may then be used to bind endotoxin and prevent its interaction with target cells.

In view of the potent immunostimulatory properties of endotoxic lipopolysaccharide, the use of monoclonal antibodies can also be extended to include their incorporation into adjuvant systems to promote immunogenicity of the host to unrelated antigens.

## EXAMPLE 3

Monoclonal antibody 3D7 as obtained in Example 2 above was assessed for its capacity to inhibit the binding of endotoxic LPS to the 80 kDa LPS receptor (see Figure 1). Increasing amounts of antibody (lanes 8-5) or an irrelevant monoclonal antibody (lanes 4-1) were added to mouse spleen cells. After 5 minutes at 37° C, $^{125}$I-ASD-LPS was added and the cells photocrosslinked by standard procedures. Solubilized cell extracts were then analyzed by SDS-PAGE and radioautographed. Control binding of LPS to the 80 kDa receptor is shown in lane 9.

## EXAMPLE 4

Inhibition of binding of monoclonal mAb 3D7 to splenocytes by LPS is shown in Figure 2. Spleen cells were bound to microtiter plates and treated with various concentrations of LPS. The cells were then washed and monoclonal antibody mAb 3D7 or an irrelevant monoclonal (anti Ia) then added. Bound antibody was assessed by ELISA.

## EXAMPLE 5

CR1 female mice, approximately six weeks of age, were taken off feed at approximately 11:30 PM the night before the experiment. At noon the following day, animals were injected intraperitoneally with mAb 5D3 or normal hamster IgG in a volume of 100 ul of sterile pyrogen free saline. One hour and 15 minutes later, all animals received 12 ng of S . enteritidis LPS endotoxin and 18 mg of D-galactosamine in $H_2O$ and were placed back on feed. Lethality was monitored hourly for the first ten hours and then at intervals for the next thirty six hours.

These experiments demonstrate that mAb pretreatment results in protection of mice against endotoxin lethality in the galactosamine sensitization model and that as little as 7.5 ug of mAb 5D3 will provide 100% protection. (See Table 4).

## EXAMPLE 6

The experimental method was exactly as described in Example 10 except that MoAb 5D3 was restricted to 15 ug and LPS doses were varied to 100 ng.

This experiment demonstrates that MoAb 5D3 at a dose of 15 ug will provide virtually complete protection against ten $LD_{100}$ challenge doses of LPS endotoxin. (See Table 5).

## EXAMPLE 7

C3Heb/FeJ female mice, approximately 4-6 months of age were pretreated with either sterile saline or with 250 ug MoAb 5D3. One hour later all mice were challenged with 250 ug of E . coli 0111 LPS endotoxin. Lethality was monitored daily for 72 hours.

The results of two experiments are shown in Table 6. These experiments provide data that mAb 5D3 will also protect normal (non-galactosamine sensitized) mice against the lethal effects of LPS endotoxin.

## EXAMPLE 8

CF1 mice 6 weeks of age (Experiment 1 of Table 7) or 10 weeks of age (Experiment 2 of Table 7) were challenged with either sterile saline, S. enteritidis LPS or mAb 5D3 (75 ug in Experiment 1, 750 ug in Experiment 2). Lethality was monitored as described in Example 5.

These experiments indicate that the endotoxin activity of mAb 5D3 as determined in the galactosamine sensitization assay is less than 0.2 ng of LPS equivalents (Experiment 1) or approximately 0.03 ng of LPS equivalents (Experiment 2) per microgram of mAb 5D3. (See Table 7).

## EXAMPLE 9

Serial dilutions of either mAb 5D3 or E . coli 0111 LPS were made in pyrogen free sterile saline. Volumes of 50 ul were placed in sterile pyrogen free glass 12 x 75 mm tubes, and 50 ul of limulus amoebocyte lysate (LAL) (provided by John L. Ryan, Yale Univ. New Haven CT) then added. Tubes were incubated at 37° C for one hour and then gelation assessed. (The Limulus lysate assay is an extremely sensitive and relatively specific test for the presence of low amounts of LPS endotoxin).

These data suggest a relative amount of endotoxin activity in the mAb 5D3 preparation of approximately 0.3 ng LPS per microgram of mAb. (See Table 8).

## EXAMPLE 10

C3HeB/FeJ Spleen cells were aseptically isolated and placed in tissue culture at a concentration of 2.5 x $10^6$ cells per ml in 96 well microtiter plates. To 200 ul of cells (in RPMI 1640 media containing glutamine and activity in the mAb 5D3 preparation of approximately 0.3 ng LPS per microgram of mAb. (See Table 8).

## EXAMPLE 10

C3HeB/FeJ Spleen cells were aseptically isolated and placed in tissue culture at a concentration of 2.5 x $10^6$ cells per ml in 96 well microtiter plates. To 200 ul of cells (in RPMI 1640 media containing glutamine and antibiotics) were added various concentra-tions of E. coli 0111 LPS and/or mAb 5D3. Cells were cultured at 37° C in a humidified 5% $CO_2$ atmosphere for 24 hours at which time 0.5 Uci of $^3H$ thymidine was added. After an additional 16 hours of culture, cells were harvested and $^3H$ thymidine incorporation determined.

These data indicate that purified mAb 5D3 is slightly mitogenic but that 50 ug/ul of mAb is less mitogenic than 16 ng/ml of LPS. Further high concentrations of mAb will partially inhibit LPS stimulation of murine lymphocytes but that inhibition is most effective at high concentrations of LPS. (See Figure 3).

## EXAMPLE 11

This is a summary of the data from Tables 7 and 8 and Figure 3. These collective results indicate the relative endotoxin activity of mAb 5D3 to be approximately 0.1 ng LPS activity per microgram of mAb 5D3. (See Table 9).

## EXAMPLE 12

CF1 mice, 6-8 weeks of age were injected intraperitoneally with either 15 ug of mAb 5D3 or various doses of S . enteritidis LPS endotoxin in a volume of 0.1 ml.

Approximately one hour later all mice were challenged with either 15 ng LPS (Experiment 1 of Table 10) or 10 ng LPS

## EXAMPLE 13

mAb 5D3 was either maintained at 4° C or heated in a boiling water bath for 60 minutes. Then serial dilutions were carried out and limulus lysate activity assessed as described in Example 8.

These data indicate that, unlike endotoxin which is stable to 100° C, the endotoxin activity of mAb 5D3 is reduced by approximately two orders of magnitude upon heating to 100° C. This experiment serves to distinguish mAb 5D3 from LPS endotoxin and further establishes that endotoxin activity observed for the mAb 5D3 preparation is actually due to mAb 5D3 and not the presence of contaminating endotoxin-LPS. (See Table 11).

## EXAMPLE 14

Individual preparations of mAb 5D3, 150 ug/ml in pyrogen free saline, were either a) maintained at 4° C, b) heated to 100° C for 60 minutes or c) supplemented with 500 ng/ml (final concentration) of E. coli 0111 LPS and then heated to 100° C for 60 minutes. A control preparation of 500 ng/ml LPS was also heated to 100° C for 60 minutes. Then 100 ul of these preparations (containing 15 ug of MoAb and/or 50 ng LPS) were injected intraperitoneally into 8 weeks old CF1 mice. After one hour and 15 minutes, 18 ng of S. enteritidis LPS and 18 mg D-galactosamine were injected intraperitoneally and survival assessed as described in Example 5.

These data indicate that the protective capacity of mAb 5D3 is destroyed by heating to 100° C whereas the protective capacity of LPS is stable at 100° C. These data confirm and extend the conclusions of Table 11 with respect to possible endotoxin contamination. (See Table 12).

## EXAMPLE 15

A highly purified preparation of the 80 kDa LPS binding protein was prepared by butanol extraction of photolabelled splenocytes from 20 C3Heb/FeJ mice. The butanol extract was further purified on eight standard isoelectric focusing gels followed by SDS-PAGE in the second dimension (the Standard O'Farrell two dimensional electrophoresis procedure described in the 1988 Journal of Immunology publications). The separated proteins were then electroblotted onto Immunobilon-P membranes and the presence of the 80 kDa protein identified by radioautography. The purified protein was then eluted in pyridine and acetonitrile, lyophilized and redissolved in phosphate buffered saline. Serial two fold dilutions of protein were prepared and used to coat 96 well microtiter plates. Reactivity to the mAb 5D3 (20 ug) or an unrelated hamster monoclonal antibody mAb 4G2 (20 ug) was then determined.

These data indicate that mAb 5D3 will bind to the purified 80 kDa LPS binding protein (See Figure 4).

## EXAMPLE 16

Rabbit (New Zealand White) were immunized with 50 ug of mAb 5D3 in Complete Freund's Adjuvant and boosted at two weeks with antigen in incomplete Freunds Adjuvant. Rabbits were then bled after an additional two weeks and the serum assessed by standard ELISA for reactivity against mAb 5D3 (Figure 5a) and the pre B cell 70Z/3 (Figure 5b).

The conclusion from these experiments is that the rabbit antiserum raised against mAb 5D3 binds with high affinity to mAb 5D3. An unexpected result was that the rabbit antiserum also bound to the 70Z/3 pre B cells. (See Figure 5).

## EXAMPLE 17

Mouse spleen cells were photolabelled with $^{125}$I-ASD-LPS as previously described. Either butanol extracts (Western blots) or whole cell extracts (immunoprecipitations) were then prepared. The butanol extracts were electrophoresed on acrylamide gels, blotted onto nitrocellulose and probed with the rabbit anti 5D3 antiserum followed by conjugated second antibody (lane A of Figure 6) or second antibody alone (lane B of Figure 6). The whole cell extracts were incubated with either rabbit anti 5D3 antiserum or with buffer and then immunoprecipitated with Igsorb. Both pellets and supernatants were the electrophoresed or polyacrylamide gels and the gels then radioautographed. Lane 1 - whole cell extract; lane 2 -supernatant of rabbit anti 5D3 plus Igsorb; lane 3 -immunoprecipitated of rabbit anti 5D3 plus Igsorb; lane 4 -supernatant of Igsorb control; lange 5 - pellet of Igsorb control.

These results establish that immunization of rabbit with mAb 5D3 can allow production of polyclonal antibody which by both Western blot analysis and specific immunoprecipitation, reacts with the 80 kDa LPS binding protein receptor. (See Figure 6).

The hybridoma cell lines secreting monoclonal antibodies 3D7 and 5D3 are on deposit at the University of Kansas Medical Center, School of Medicine, Department of Microbiology, Molecular Genetics and Immunology, 39th and Rainbow Blvd., Kansas City, Kansas 66103 and since May 4, 1989 (3D7 and 5D3) at the American Type Culture Collection (ATCC) 12301 Parklawn Drive, Rockville, MD 20852 under the designations as follows: 3D7 (HB10124) and 5D3 (HB10125).

## EXAMPLE 18

Experiments were performed to determine the effect of mAb 5D3, alone and in combination with other materials, as an inducer of macrophage mediated tumor cytotoxicity.

Bone marrow cells were obtained from male C3H/HeN and C3H/HeJ mice, 6-9 weeks old. Bone marrow was removed from the tibiae and femora of the mice, and these were cultured on bacterial grade Petri dishes, following Leung et al., Cell Tissue Res. 239 : 693 (1985), the disclosure of which is incorporated by reference. Macrophages from 14-18 day old cultures were used in experiments.

Non-adherent cells were removed with supernatant media of the cultures. Loosely adherent cells were incubated at 4° C in PBS for 20 minutes, followed by detachment with a rubber policeman. Following this approximately $6x10^4$ macrophages were seeded per well in 96 well culture plates.

The cells were permitted to adhere in H-MEM containing 10% FBS for 2 hours at 37° C, in a 5% $CO_2$ atmosphere. This produced macrophage monolayers which were washed twice with fresh H-MEM containing 10% FBS immediately before use.

Duplicate samples of the layered cells were then treated for 6 hours with 100 ul of a stimulus diluted in H-MEM containing 10% FBS. Specifically, various concentrations of mAb 5D3, alone and with 3U/ml of IFN-$\gamma$ were used, as were various concentrations of E. coli 0111:$B_4$ LPS.

Following the incubation with stimulus, $2x10^4$ $^{51}$Cr labelled P815 mastocytoma cells were seeded into each well, in 100 ul of medium. Sixteen hours later, the uppermost 0.1 ml of supernatant medium was gently aspirated from each well, and assayed for radio-activity in a gamma spectrometer. The release of $^{51}$Cr into medium is a measurement of cell death, as per Russell, in Adams et al., ed., Methods For Studying Mononuclear Phagocytes (Academic Press, N.Y., 1980), p. 793. The results are expressed by the formula:

$$\%\ \text{specific}\ ^{51}\text{Cr release} = \frac{\text{Experimental Release-Spontaneous Release}}{\text{Total release counts - spontaneous release}} \times 100$$

"Total release counts" is defined at the number released from Target cells which had been frozen and thawed in hypotonic medium. "Spontaneous release" is defined as the $^{51}$Cr released from target cells cultured on monolayers of unstimulated monolayers.

The results are shown in the various panels of Figure 7. In figure 7a, the mAb alone activated cytotoxic macrophages up to about 60% release possible. When IFN-$\gamma$ was added, as shown in Figure 7b, the activity of the mAb was potentiated, as evidenced by the increase to 60% toxicity at a lower concentration of mAb. In comparative experiments not shown here, the use of IFN-$\gamma$ by itself, at the same concentration, had no effect.

The expected effect of LPS was manifested, as shown in Figure 7c.

## EXAMPLE 19

Earlier work had shown that macrophages from C3H/HeJ mice, while relatively refractory to LPS stimulation, can be immunostimulated with, e.g., heat killed Listeria monocytogenes ("HKLM"). See, e.g., Ruco et al., J. Immunol 120 : 329 (1978). To that end, experiments were carried out to determine the effect of mAb 5D3 on induction of tumor cell killing activity in both control (C3H/HeN) responder and low responder (C3H/HeJ) bone marrow derived macrophages. The protocol generally followed that of Example 18, with HeN cells being used for control responder, and HeJ as low responder. The mAb 5D3, LPS, and HKLM were combined with 10 u/ml of murine IFN-γ, and $^{51}$Cr release after 6 hours was measured. The results, depicted in Figure 8, show that indeed, those macrophages not sensitive to LPS were also not sensitive to mAb 5D3, i.e., there was no significant stimulation of tumoricidal activity in HeJ cells.

## EXAMPLE 20

Given that the mAb 5D3 showed behavior that is qualitatively similar to that of LPS, it was necessary to show that the observed mAb activity was not a result of contaminating endotoxin similar to concerns addressed in Examples 13 and 14. In this connection, the endotoxin is known to preserve its biological activity for the most part after prolonged heating at 100° C, while antibodies of course, generally do not. This example describes how contamination with endotoxin was essentially ruled out. The results should be compared with those described in the above cited examples.

Various concentrations of either unheated or heated (100° C, 1 hours) mAb 5D3 were used with the above mentioned HeN cells, in the presence of 3 U/ml IFN-γ. These results, shown in panel 9a, parallel the previous experiments, i.e., the test for cytotoxicity is as described supra . Heated material showed no activity, suggesting no endotoxin contamination.

## EXAMPLE 21

To further prove that endotoxin contamination did not occur, experiments were carried out using polymyxin B, known to be a potent inhibitor of LPS activity on macrophages. See Chio et al., J. Infect. Dis. 159 : 872 (1989), Stokes et al., J. Infect. Dis. 160 : 52 (1989) Morrison et al., Immunochem 13: 813 (1976).

In these experiments, mAb 5D3 was co-incubated with polymyxin B, again using protocols and parameters set forth in the previous examples. As shown in Figure 9B, the stimulating activity was inhibited only marginally. In contrast, when LPS was mixed with the same amount of polymixin B, its activity was abolished completely. Indeed, when even 1 ug/ml of polymixin B was used with 10 ng/ml LPS, activity dropped from 58% to 1.3% $^{51}$Cr release. Thus, mAb 5D3 activity is heat labile and polymixin B insensitive, and does not result from contamination with endotoxin.

## EXAMPLE 22

The effect of LPS on mAb 5D3's activity was tested. Macrophages were primed with IFN-γ, and then with heated or unheated mAb 5D3, in the presence or absence of a subthreshhold triggering concentration of LPS (0.1 ng/ml). These results are shown in Figure 10. The C3H/HeN macrophages were tested, again as described in the previous experiments.

It will be seen that mAb below certain concentration of 5D3 the addition of 0.1 ng/ml of LPS increased activity by almost threefold. This did not occur at concentrations above about 7 ug/ml. The heated mAb 5D3 did not show any activity, nor did the addition of LPS change this.

These results show that the combination of LPS and mAb 5D3 may have a somewhat more than additive result.

## EXAMPLE 23

Previous work has shown that anti IFN-$\alpha/\beta$ in culture medium inhibits LPS induced macrophage tumoricidal activity. See Leu et al., Immunobiology 171 : 220 (1986). This has been shown to result from abrogation of the autocrine/paracrine priming effect of IFN-$\alpha/\beta$ initiated by LPS-stimulated macrophages. To determine the effect of anti-IFN-$\alpha/\beta$ antibody on the effect of mAb 5D3, C3H/HeN macrophage monolayers were stimulated in the presence of either of sheep anti-IFN-$\alpha/\beta$ antibody or normal sheep IgG. Again, reference is made to the assays described supra for how the activity was tested. Figure 11 shows that the anti-IFN-$\alpha/\beta$ antibody had the same effect on 5D3 as it did on LPS.

Examples 18-23 show unequivocably that the 80 kilodalton membrane LPS binding protein receptor, which mAb 5D3 recognizes, is at least one of the entities by which activation for tumor cell killing is regulated.

Mab 5D3 faithfully reproduces the activity of LPS with respect to mediation of tumor cytotoxicty by macrophages, but does not have the same toxic effect. As such, it can be said that the mAb which specifically binds to the 80 kilodalton LPS receptor via its protein component is an agonist signal for macrophage activation.

These results show that mAb 5D3, a mAb which binds to the protein component of the aforementioned LPS receptor, stimulates macrophages into cytotoxic effect against tumor cells. It is effective in combination with IFN-$\gamma$ as well, although the interferon is not a necessary material for provoking the effect.

It has been shown, supra from earlier experiments, that mAb 5D3 is not toxic to its host. Thus, the experiments described herein would be expected to be transferrable to in vivo therapy, as the model used herein is predictive of this effect.

The foregoing thus provides a method for provoking a cytotoxic effect by macrophages against tumor cells, such as mastocytoma cells, by administering to a subject in need of the treatment a therapeutically effective amount of a monoclonal antibody which specifically binds to the protein portion of the 80 kilodalton LPS glycoprotein receptor, such as mAb 5D3. The monoclonal antibody can be administered together with gamma interferon, but this is not a requirement.

The amount of the monoclonal antibody, and the gamma interferon will vary depending upon the subject so treated. As has been shown, supra, when the mAb is added alone, a concentration of about 10 ug/ml of monoclonal antibody is the preferred minimum concentration. When administered with IFN-$\gamma$ at a concentration of about 3U/ml, the concentration of mAb can be decreased to as little as 3 ug/ml. In an especially preferred embodiment, a concentration of about 10 ug/ml of the mAb, and the 3U/ml of IFN-$\gamma$ are administered together.

In connection with this, a therapeutic composition of a monoclonal antibody as described supra , and IFN-$\gamma$ is also described. The mAb is present in an amount sufficient to cause activation of macrophages against tumor cells, while the INF-$\gamma$ is present in an amount sufficient to potentiate the effect of the monoclonal antibody.

The terms and expressions which have been employed are used as terms of description and not of limitation, and there is no intention in the use of such terms and expressions of excluding any equivalents of the features shown and described or portions thereof, it being recognized that various modifications are possible within the scope of the invention.

Table 1

| Amino Acid Composition of LPS-Binding Protein[1] | |
|---|---|
| Amino Acid[2] | #Residues[2] |
| Asp | 73 |
| Glu | 105 |
| Ser | 45 |
| Gly | 46 |
| His | 19 |
| Arg | 33 |
| Thre | 39 |
| Ala | 53 |
| Pro | 32 |
| Tyr | 27 |
| Val | 41 |
| Met | 5 |
| Ileu | 16 |
| Leu | 68 |
| Phe | 31 |
| Lys | 81 |

[1]LPS binding protein purified from two-dimensional gels, blotted onto Immobilon and subject to acid hydrolysis and amino acid analysis.
[2]Average of three independent determinations.
[3]Based upon 80,000 kDa molecular mass.

Table 2

| Effect of Chemical Modification on Monoclonal Antibody Binding to Purified 80 kDa LPS Receptor | | |
|---|---|---|
| | Absorbance at 450nm with MoAb: | |
| Treatment[1] | 3 D 7 | 5 D 3 |
| None | 0.31 | 0.16 |
| Periodate 1mM | <0.01 | 0.23 |
| 10mM | <0.01 | 0.67 |
| Protease K 0.1ug/ml | 0.29 | <0.01 |
| 1.0ug/ml | 0.28 | <0.01 |

[1]Microtiter plates coated with 80 kDa LPS Receptor treated with periodate or protease K for 1.0 hour at 37° C.

Table 3

Protection of Mice Against Endotoxin Lethality
with Purified 5D3 Monoclonal

| Pretreatment[1] | Challenge | Survivors @ 18 hours |
|---|---|---|
| saline | 0.01μg LPS | 1/10 (10%) |
| 5D3 (75μg) | 0.01μg LPS | 10/10 (100%) |

[1]Mice (CF1 females, 18-22gm) were administed either pyrogen free saline or purified 5D3 monoclonal antibody intraperitoneally in a final volume of 60μl. One hour later mice received a second intraperitoneal injection of S. enteritidis LPS and 18 mg of D-galactosamine. Survivors were monitored hourly for the first 9 hours and then at 12 hour intervals. All control mice died within 6-12 hours. No mice given 5D3 died even after 48 hours.

TABLE 4

| Protective Efficacy of MoAb 5D3 in Galactosamine Treated Mice | | | | |
|---|---|---|---|---|
| | | Survivors/Total | | |
| Pretreatment | Challenge[1] | Expt 1 | Expt 2 | Total (%) |
| saline | 12ng LPS | 2/15 | 8/16 | 10/31 (34%) |
| 75ug MoAb 5D3 | 12ng LPS | 10/10 | 10/10 | 20/20 (100%) |
| 7.5ug MoAb 5D3 | 12ng LPS | - | 10/10 | 10/10 (100%) |
| 0.75ug MoAb 5D3 | 12ng LPS | - | 3/5 | 3/5 (60%) |
| 75ug IgG | 12ng LPS | - | 4/10 | 4/10 (40%) |
| --- | saline | 5/5 | 5/5 | 10/10 (100%) |

[1]All mice given a simultaneous dose of 18mg D-galactosamine

TABLE 5

| Protective Efficacy of MoAb 5D3 in Galactosamine Treated Mice | | |
|---|---|---|
| Effect of LPS Challenge Dose | | |
| Pretreatment | Challenge[1] | Survivors/Total |
| saline | 10ng LPS | 0/8 |
| 15ug MoAb 5D3 | 10ng LPS | 8/8 |
| 15ug MoAb 5D3 | 3ng LPS | 8/8 |
| 15ug MoAb 5D3 | 100ng LPS | 7/8 |

[1]All mice given a simultaneous dose of 18mg D-galactosamine.

TABLE 6

| Protective Efficacy of MoAb 5D3 in Normal C3HeB/FeJ | | | | |
|---|---|---|---|---|
| Pretreatment | Challenge | EXP1 | EXP2 | TOTALS |
| saline | 250ug LPS | 4/9 | 7/12 | 11/21(53%) |
| | | P~0.1 | | |
| 250ug MoAb 5D3 | 250ug LPS | 8/9 | 11/11 | 19/20(95%) |

TABLE 8

| Assessment of Endotoxin Activity of MoAb 5D3 | | | | | | | |
|---|---|---|---|---|---|---|---|
| The Limulus Amoebocyte Lysate Assay Method | | | | | | | |
| Gelation* | | | | | | | |
| Amount: | 15ng | 3ng | 600pg | 100pg | 10pg | 1pg | 0.1pg |
| MoAb5D3 | + + | + | - | - | - | - | - |
| LPS | + + | + + | + + | + + | + + | + | - |

*Scale Solid Gel ( + + ), Flocculant ppt ( + ), None (-)
Endotoxin Equivalents: 0.3ng "LPS"/ug

TABLE 7

| Assessment of Endotoxin Activity of MoAb 5D3 | | | |
|---|---|---|---|
| This Galactosamine Sensitized Mouse Model[1] | | | |
| | Challenge Dose | Survival | Endotoxin Equivalents |
| Expt 1 | saline<br>15ng LPS<br>75ug 5D3 | 6/6<br>1/5<br>6/6 | --<br>--<br><0.2ng "LPS"/ug MoAb |
| Expt 2 | saline<br>20ng LPS<br>750ug 5D3 | 8/8<br>2/8<br>2/8 | --<br>--<br>~0.03ng "LPS"/ug MoAb |

[1]All mice given a simultaneous dose of 18mg D-galactosamine.

TABLE 9

| Estimations of Endotoxin Activity of MoAb 5D3 | | |
|---|---|---|
| Experiment # | Method | Level of Activity<br>ng "LPS"/ug MoAb 5D3 |
| 1 | Galactosamine Lethality | <0.2ng |
| 2 | Galactosamine Lethality | ~0.03ng |
| 3 | Limulus (unheated) | ~0.3ng |
| 4 | Limulus (unheated) | <0.2ng |
| 5 | Mitogen | <0.2ng |
| Estimated average ~0.ing "LPS"/ug MoAb 5D3 | | |

TABLE 10

| Effect of LPS Pretreatment on LPS Lethality in Galactosamine Sensitized Mice | | |
|---|---|---|
| Experiment 1 | | |
| Pretreatment | Challenge* | Survivors/Total |
| saline | 15ng LPS | 1/5 |
| 15ug MoAb 5D3 | 15ng LPS | 5/5 |
| 100ng LPS | 15ng LPS | 4/6 |
| Experiment 2 | | |
| Pretreatment | Challenge* | Survivors/Total |
| saline | 10ng LPS | 0/8 |
| 15ug MoAb 5D3 | 10ng LPS | 8/8 |
| 1.0ng LPS | 10ng LPS | 2/8 |
| 3.0ng LPS | 10ng LPS | 4/8 |
| 10ng LPS | 10ng LPS | 5/8 |
| 30ng LPS | 10ng LPS | 8/8 |

*All mice given a simultaneous dose of 18mg of D-galactosamine

**Claims**

1. Method for stimulating macrophages to generate a cytotoxic effect against a tumor, comprising administering to a host with a tumor an amount of a therapeutic composition containing a monoclonal antibody which specifically binds to the protein portion of the 80 kilodalton, glycoprotein receptor for bacterial lipopolysaccharide endotoxin sufficient to stimulate cytotoxicity of said macrophages against said tumor.

2. Method of claim 1, wherein said monoclonal antibody is 5D3.

3. Method of claim 1, wherein said monoclonal antibody is present in said therapeutic composition at a concentration of at least about 10 ug/ml.

4. Method of claim 1, wherein said therapeutic composition further comprises gamma interferon.

5. Method of claim 4, wherein said gamma interferon is present in an amount of at least about 3U/ml.

6. Method of claim 4, wherein said therapeutic composition contains said monoclonal antibody at a concentration of at least about 3ug/ml.

7. Method of claim 1, wherein said tumor is a mastocytoma.

8. Composition useful in stimulating cytotoxic effect of macrophages against tumors, comprising an amount of a monoclonal antibody which specifically binds to the protein portion of the 80 kilodalton glycoprotein receptor for bacterial lipopolysaccharide endotoxin sufficient to stimulate macrophage cytotoxicity against said tumor and an amount of gamma interferon sufficient to potentiate the effect of said monoclonal antibody on macrophages.

9. Composition of claim 8, wherein said monoclonal antibody is 5D3.

10. Composition of claim 4, wherein said monoclonal antibody is present at a concentration of at least 3 ug/ml.

11. Composition of claim 8, wherein said gamma interferon is present at a concentration of at least 3U/ml.

12. Method for treating bacterial lipopolysaccharide endotoxin caused septic shock in mammals comprising administering one or more antibodies specific for bacterial lipopolysaccharide endotoxin-binding receptor on mammalian cells to counter the effects of the lipopolysaccharide on said cells.

13. Method of claim 12, wherein said one or more antibodies is a monoclonal antibody.

14. Method of claim 13, wherein said monoclonal antibody is SD3 or 3D7.

15. Method of claim 12, wherein said one or more antibodies comprise polyclonal antibodies.

16. Method for treating bacterial lipopolysaccharide endotoxin caused septic shock in mammals comprising administering one or more antibodies generated in response to immunization with a monoclonal antibody specific for bacterial lipopolysaccharide endotoxin binding receptor on mammalian cells to counter the effects of the lipopolysaccharide on the cells.

17. Method of claim !6, wherein said monoclonal antibody is 5D3 or 3D7.

18. Polyclonal antibody which specifically binds to bacterial lipopolysaccharide endotoxin binding receptor of mammalian cells.

19. Purified mammalian glycoprotein binding receptor for bacterial lipopolysaccharide endotoxin having a molecular weight of 80 kilodaltons.

20. Monoclonal antibody which specifically binds to bacterial lipopolysaccharide endotoxin binding receptor on mammalian cells.

21. Monoclonal antibody of claim 20 which binds to a carbohydrate mciety of said receptor.

22. Monoclonal antibody of claim 20 which binds to a protein moiety of said receptor.

23. Monoclonal antibody of claim 20, selected from the group consisting of 3D7 and 5D3.

24. Hybridoma cell line which produces the monoclonal antibody of claim 20.

25. Hybridoma cell line which produces the monoclonal antibody of claim 24.

filed

FIG. I.

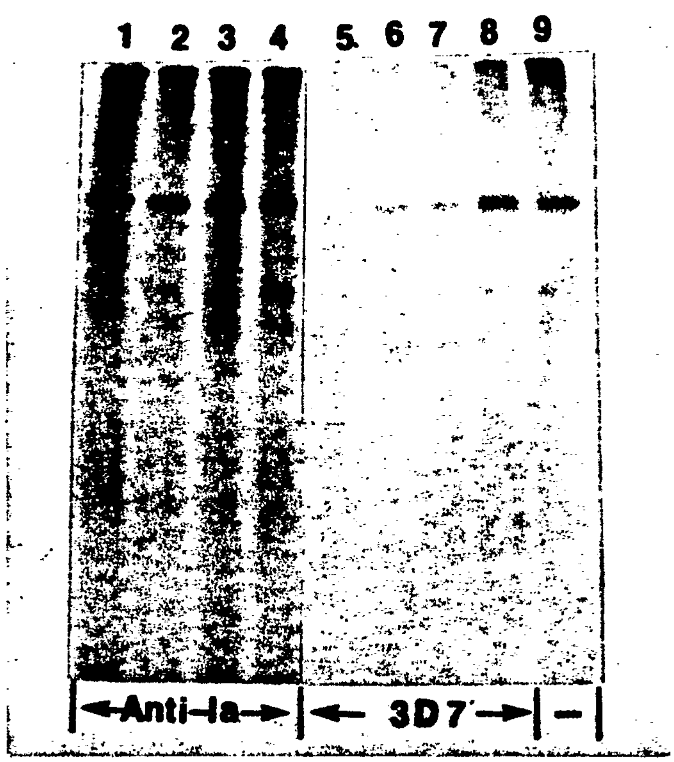

Nouvellement déposé / Newly filed

# FIG. 2.

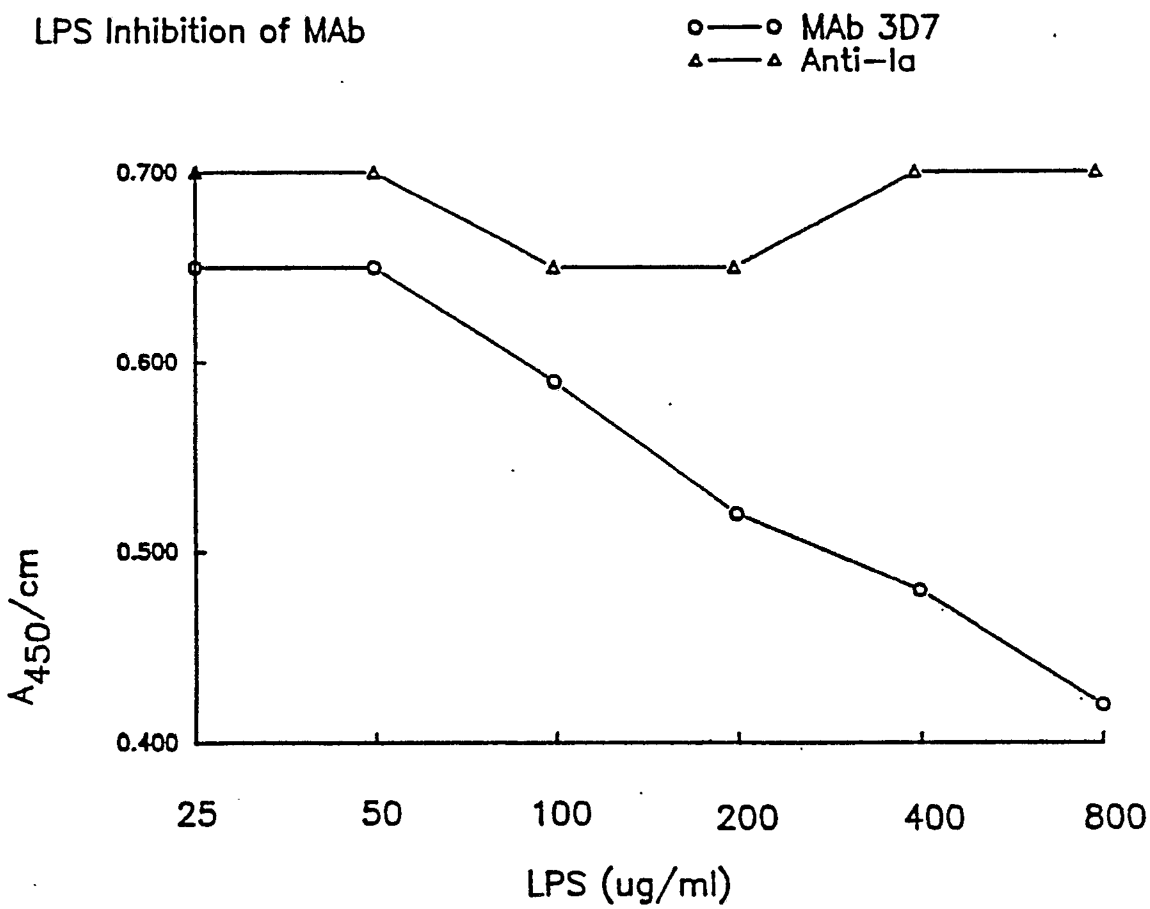

... filed
... déposé

# FIG. 3.

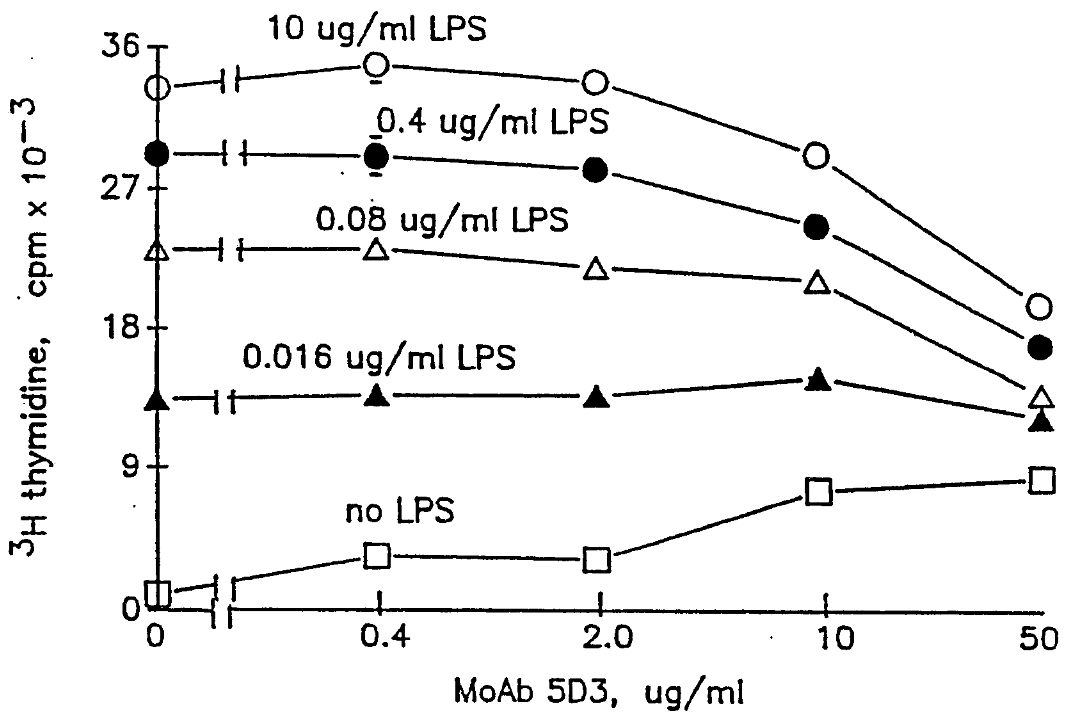

Neu eingereicht / Newly filed
Nouvellement déposé

# FIG. 4.

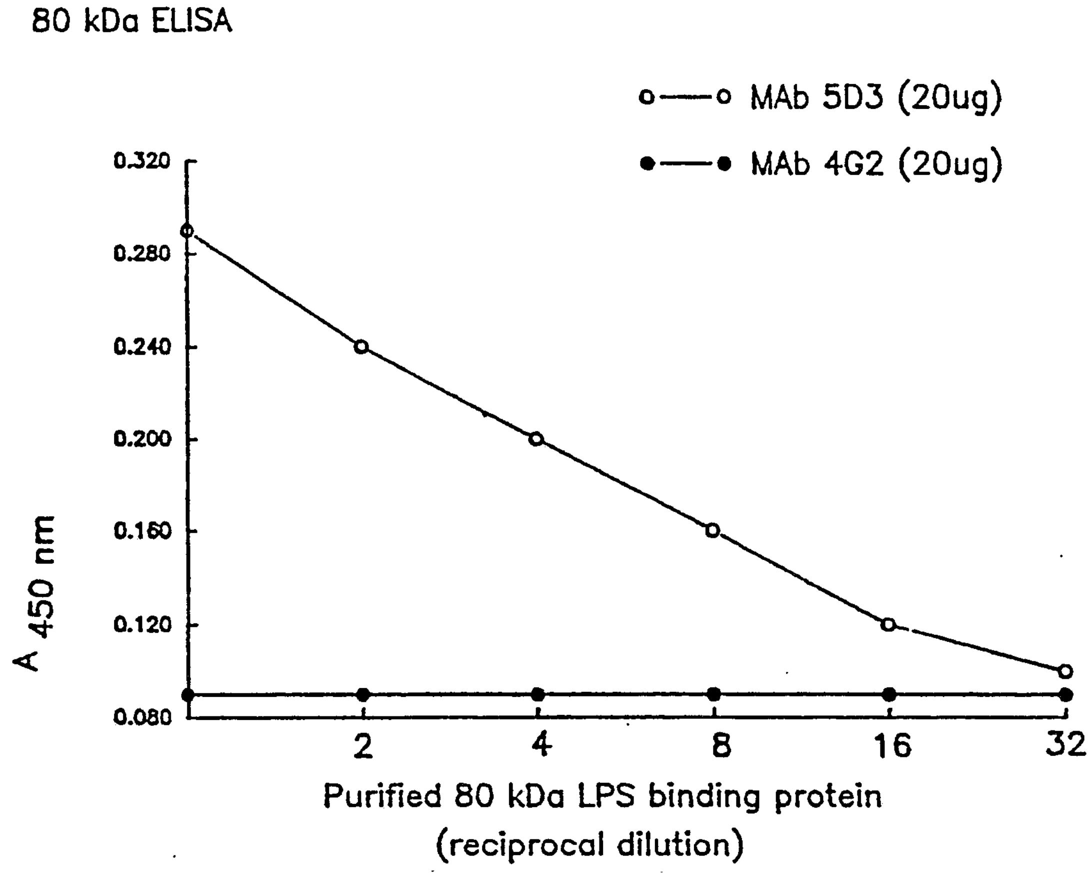

**FIG. 5a.**

Rabbit serum activity against MoAb 5D3.

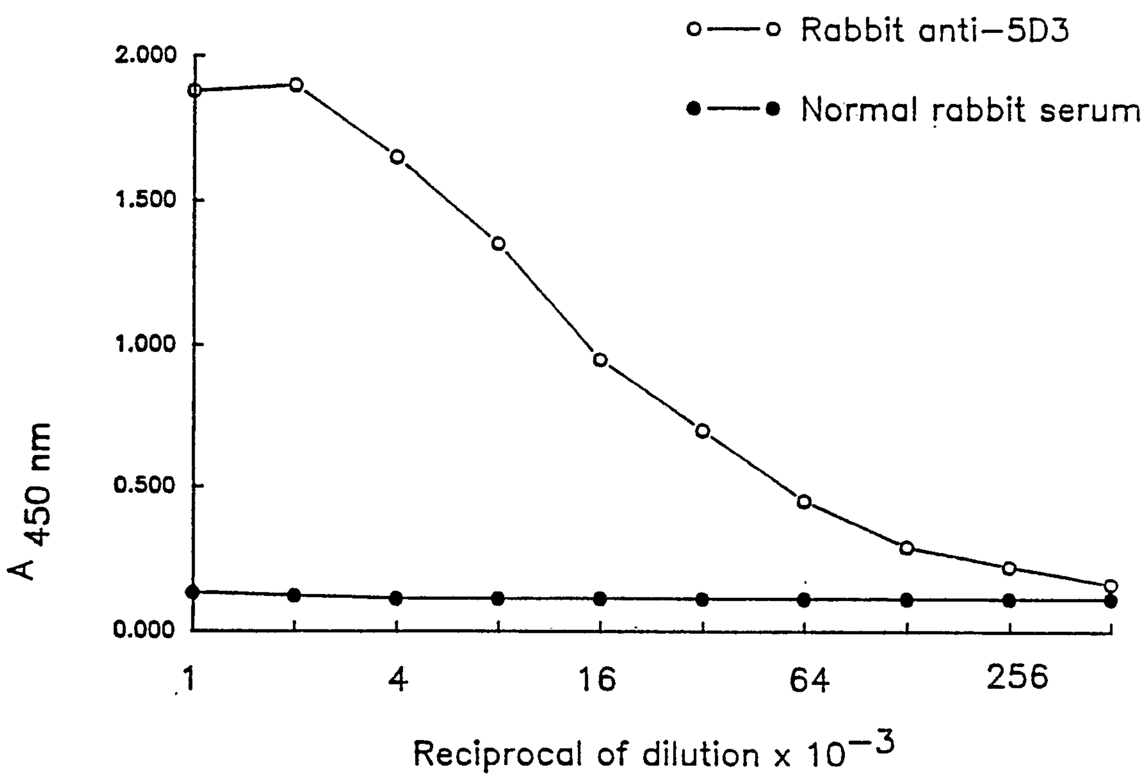

# FIG. 5b.

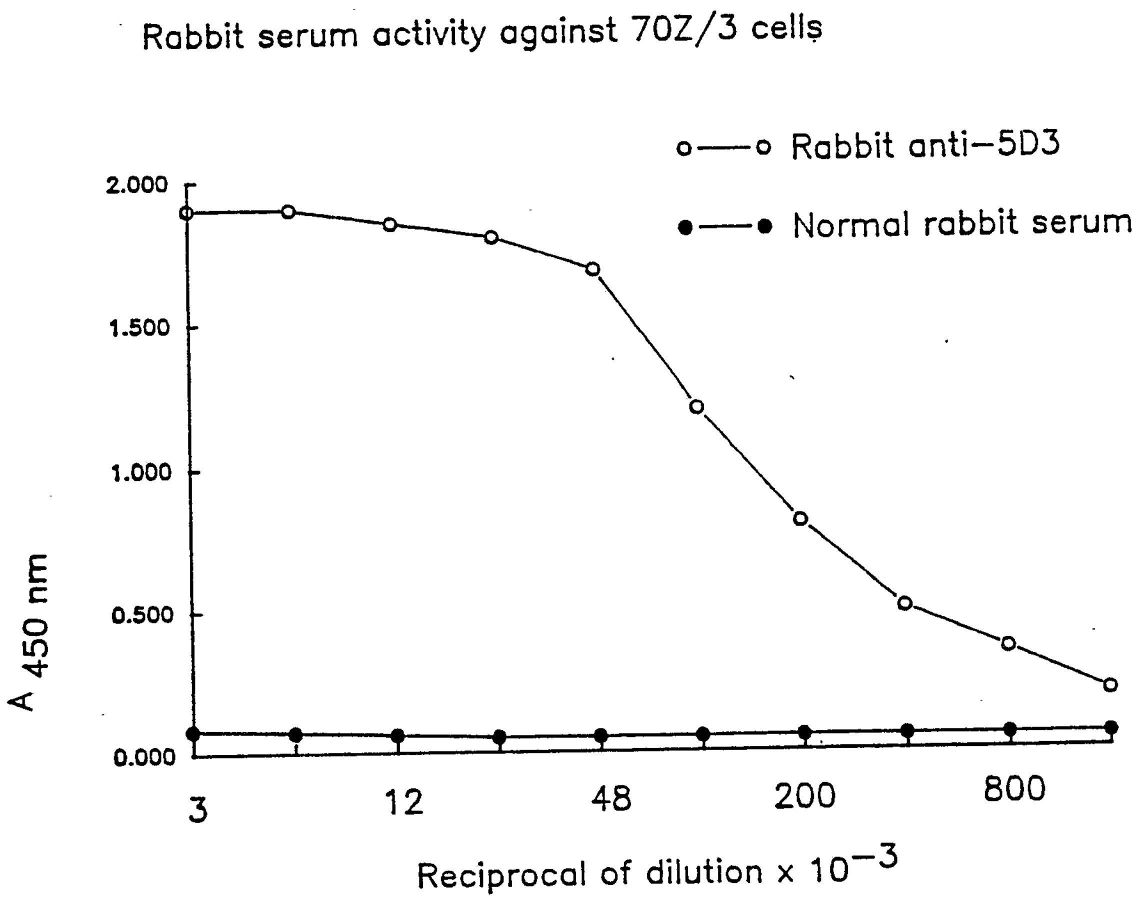
Rabbit serum activity against 70Z/3 cells
Rabbit anti–5D3
Normal rabbit serum
2.000
1.500
1.000
0.500
0.000
A 450 nm
3
12
48
200
800
Reciprocal of dilution x $10^{-3}$

Neu eingereicht / Newly filed
Nouvellement déposé

FIG. 6.

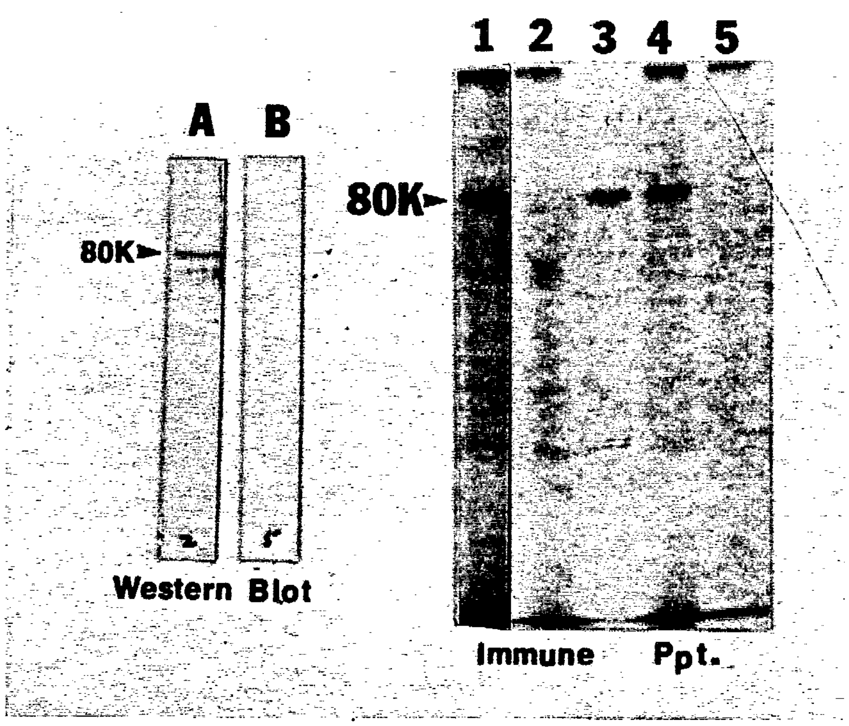

# FIG. 7A.

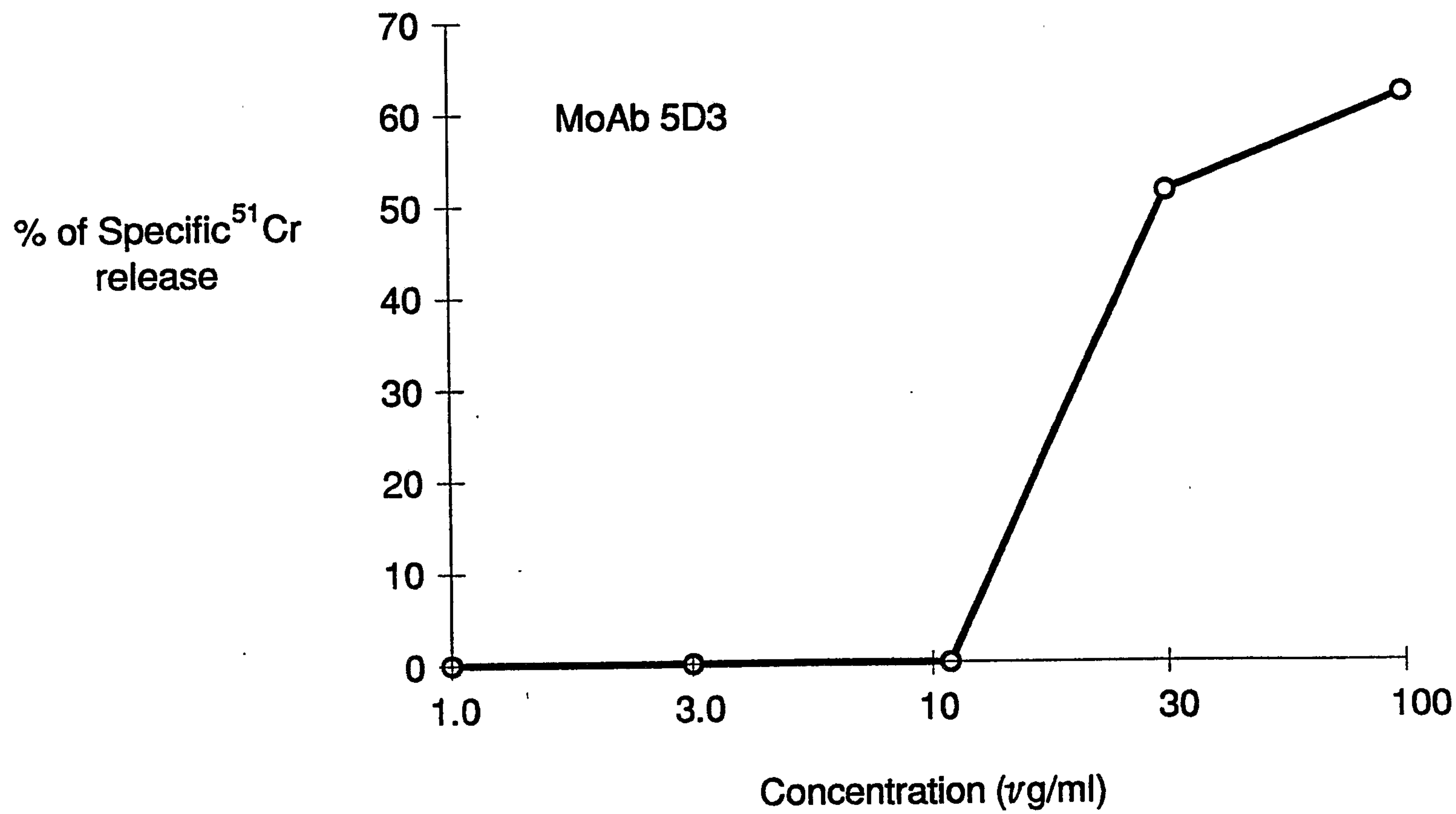
MoAb 5D3
% of Specific$^{51}$Cr release
70
60
50
40
30
20
10
0
1.0
3.0
10
30
100
Concentration (νg/ml)

FIG. 7B.

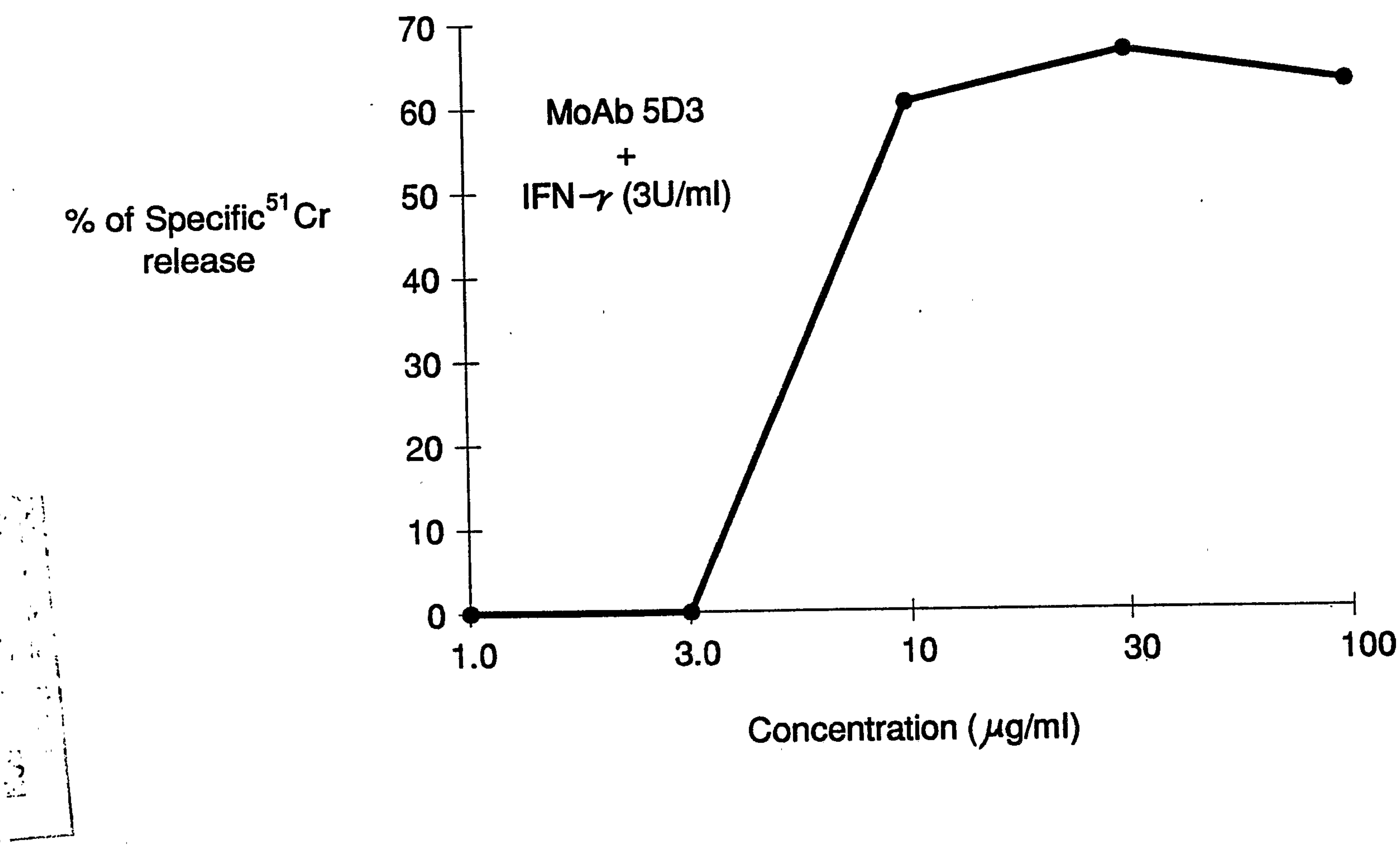
70
60
50
40
30
20
10
0
% of Specific 51 Cr release
MoAb 5D3
+
IFN-γ (3U/ml)
1.0
3.0
10
30
100
Concentration (μg/ml)

FIG. 7C.

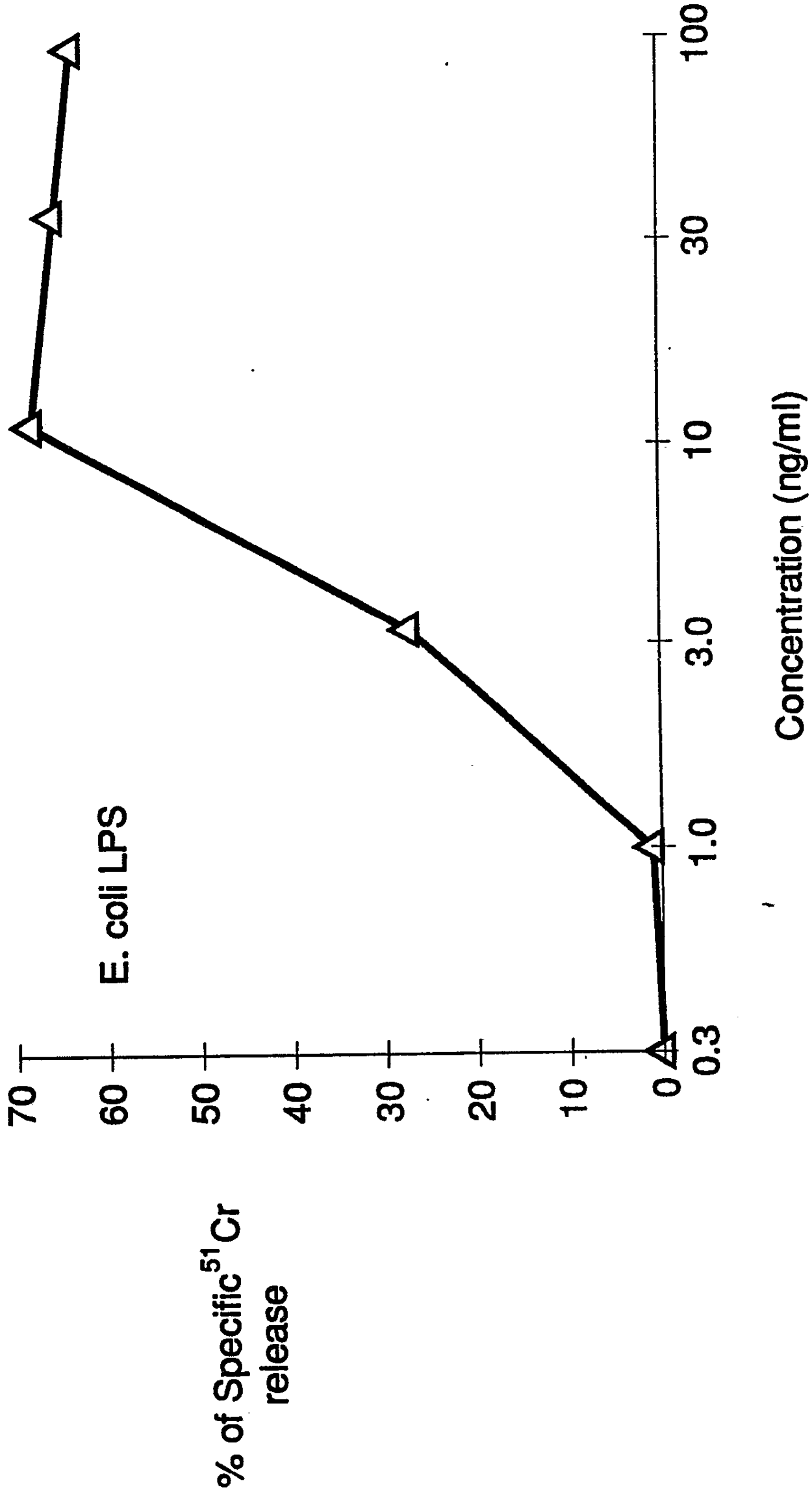
E. coli LPS
% of Specific $^{51}$Cr release
70
60
50
40
30
20
10
0
0.3
1.0
3.0
10
30
100
Concentration (ng/ml)

FIG. 8.

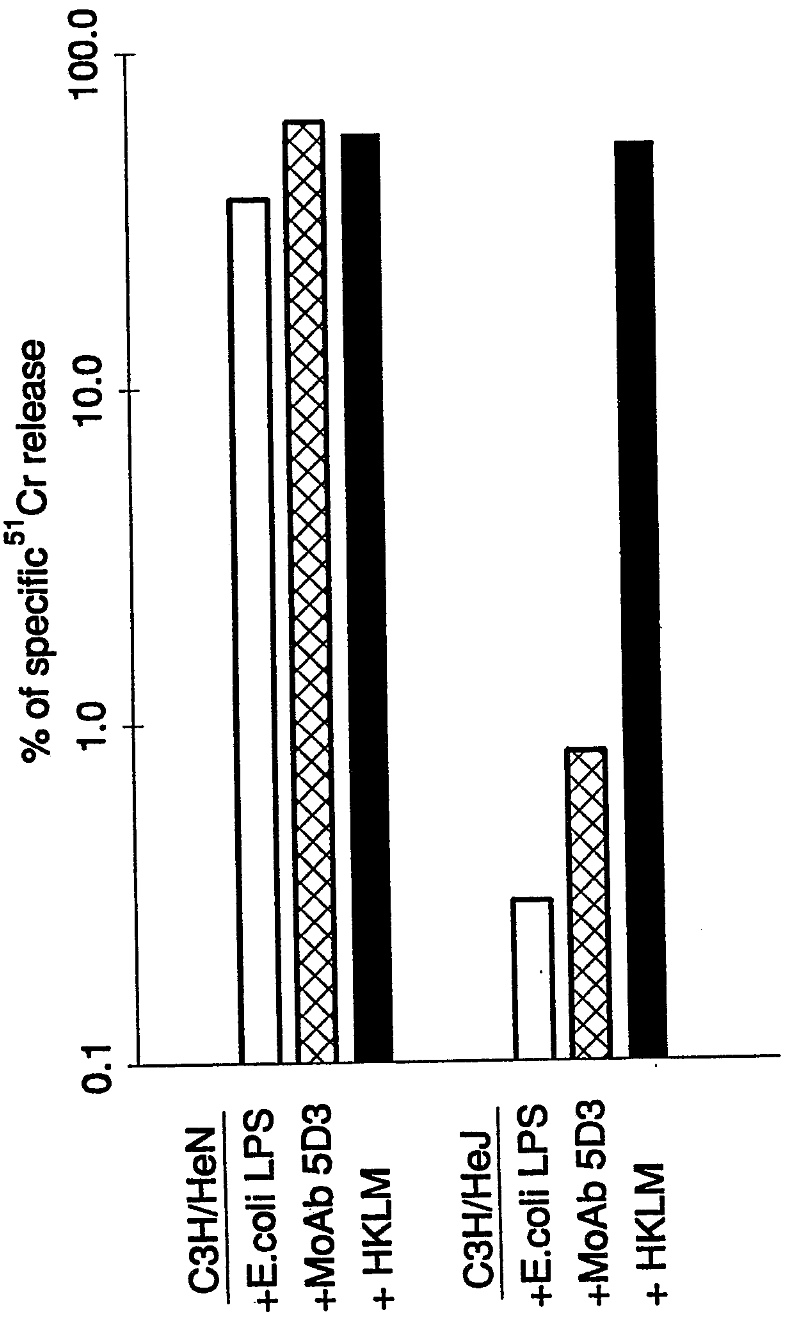

% of specific $^{51}$Cr release
0.1
1.0
10.0
100.0
C3H/HeN
+E.coli LPS
+MoAb 5D3
+ HKLM
C3H/HeJ
+E.coli LPS
+MoAb 5D3
+ HKLM

Neu eingereicht / Newly filed
Nouvellement déposé



# FIG. 9.

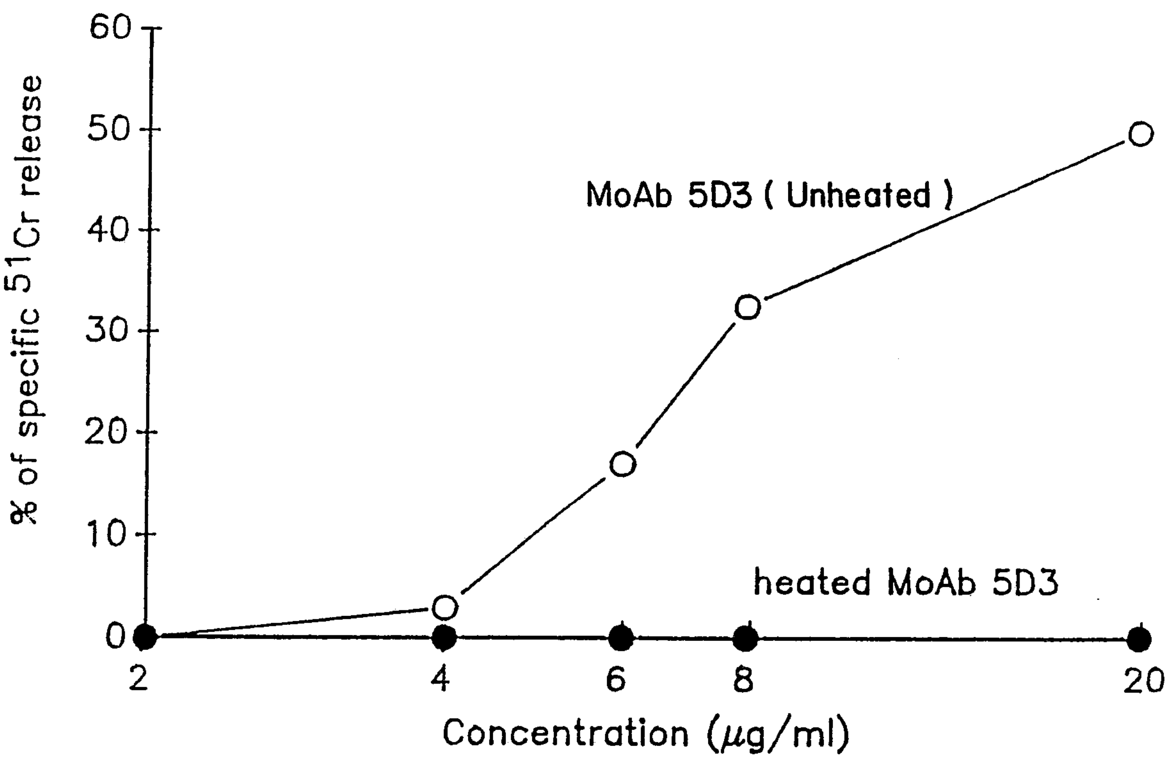

# FIG. 10a.

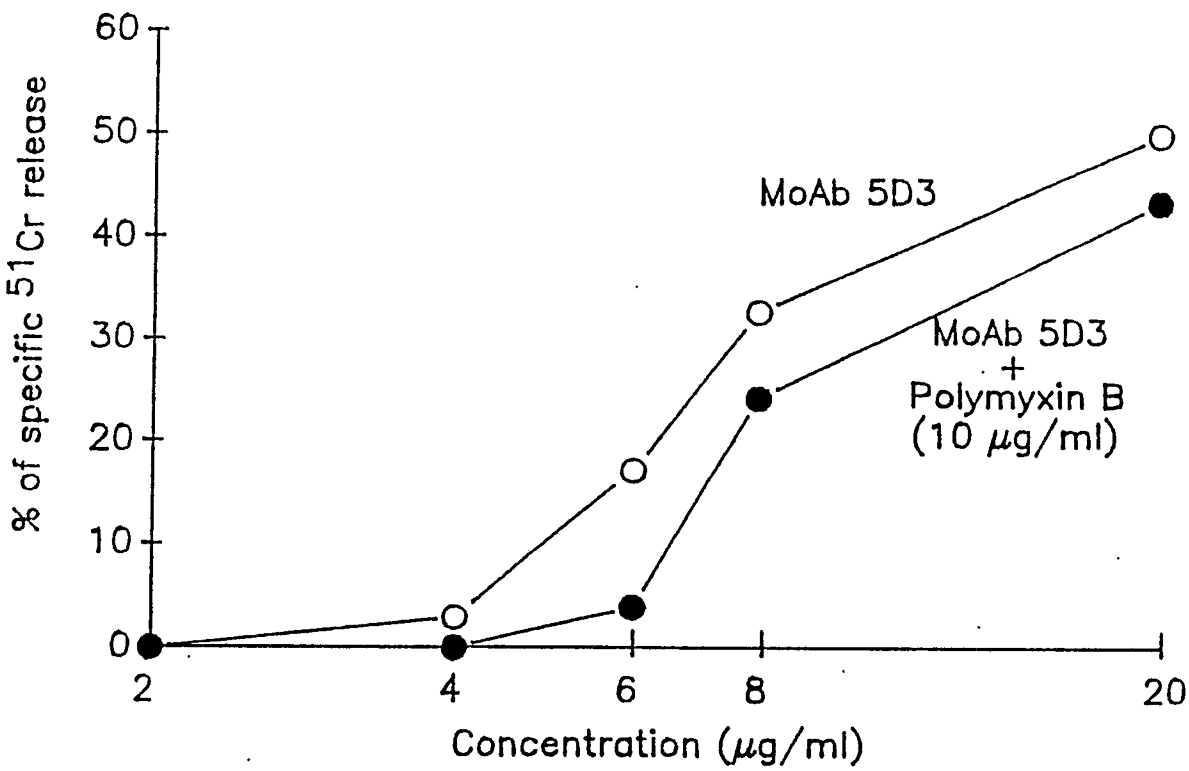
60
50
40
30
20
10
0
% of specific 51Cr release
MoAb 5D3
MoAb 5D3
+
Polymyxin B
(10 µg/ml)
2
4
6
8
20
Concentration (µg/ml)

FIG. 10b.

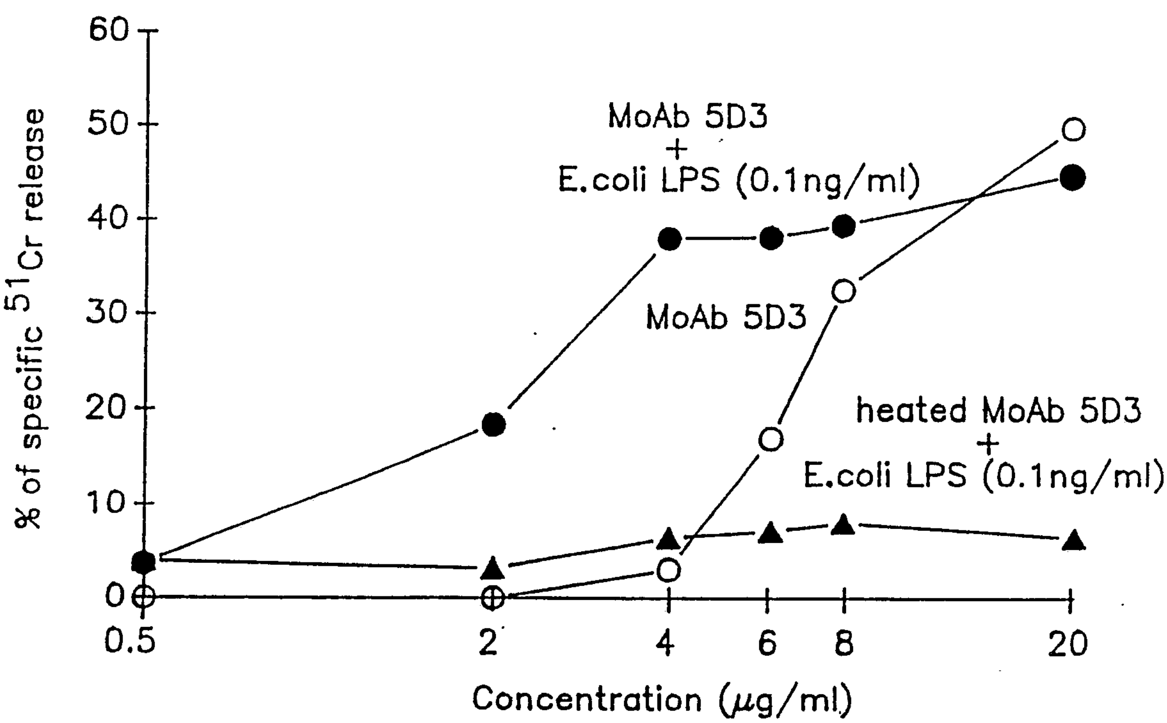
60
50
40
30
20
10
0
% of specific 51Cr release
MoAb 5D3
+
E.coli LPS (0.1ng/ml)
MoAb 5D3
heated MoAb 5D3
+
E.coli LPS (0.1ng/ml)
0.5
2
4
6
8
20
Concentration (μg/ml)

FIG. 11.

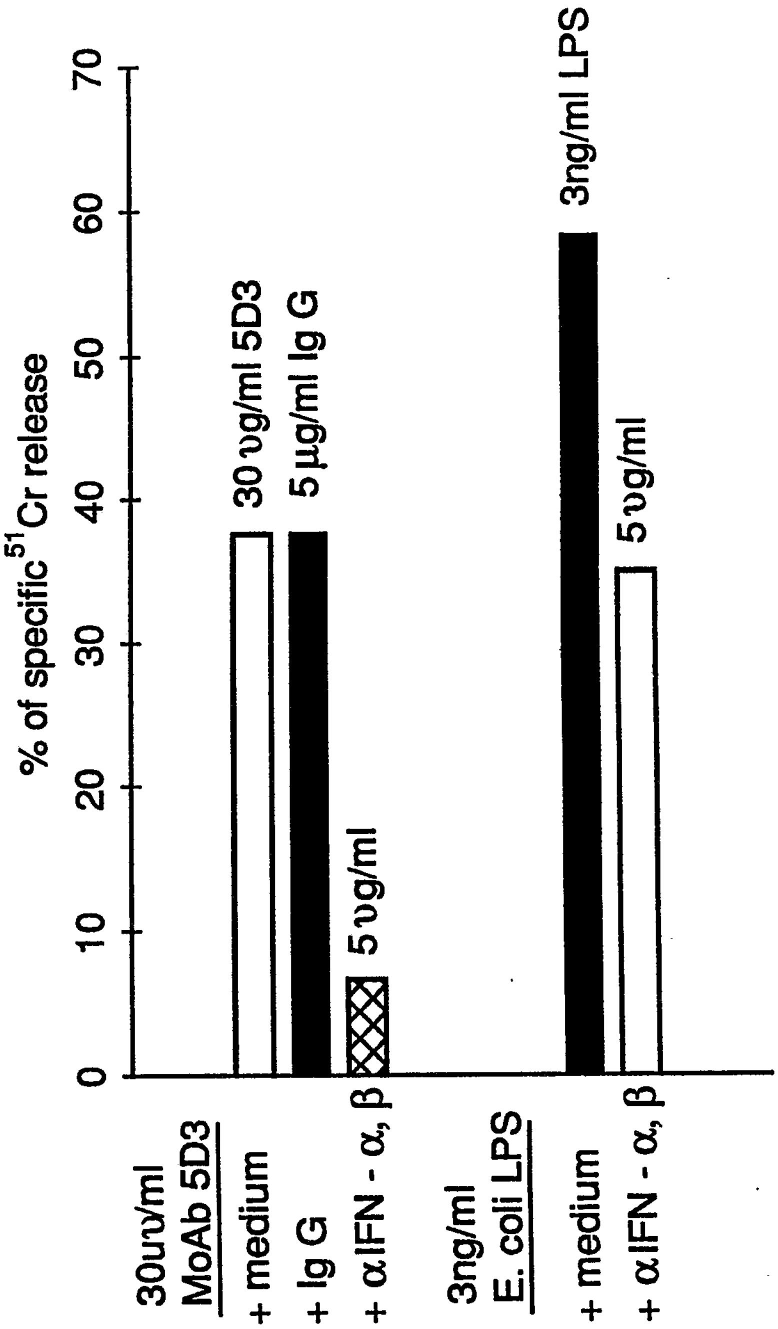

% of specific 51Cr release
0
10
20
30
40
50
60
70
30uυ/ml
MoAb 5D3
+ medium
+ Ig G
+ αIFN - α, β
3ng/ml
E. coli LPS
+ medium
+ αIFN - α, β
30 υg/ml 5D3
5 µg/ml Ig G
5 υg/ml
3ng/ml LPS
5 υg/ml